# EUROPEAN PATENT SPECIFICATION

(11) **EP 4 397 326 B1**
(45) Date of publication and mention of the grant of the patent: **17.06.2026**
(21) Application number: 23020544.5
(22) Date of filing: 08.12.2023
(51) Int. Cl.: A61L 15/28, A61L 15/46, A61L 26/00

(54) **METHOD OF PREPARING KEFIRAN FOIL AND KEFIRAN FOIL**
VERFAHREN ZUR HERSTELLUNG VON KEFIRANFOLIE UND KEFIRANFOLIE
PROCÉDÉ DE PRÉPARATION DE FEUILLE DE KEFIRAN ET FEUILLE DE KEFIRAN

(30) Priority: 09.12.2022 PL 44310422
(43) Date of publication of application: 10.07.2024
(73) Proprietor: Uniwersytet Mikolaja Kopernika w Toruniu, 87-100 Torun (PL)
(72) Inventor: Chelminiak-Dudkiewicz, Dorota, 87-100 Torun (PL); Ziegler-Borowska, Marta, 87-100 Torun (PL)

(56) References cited:
- CA-A1- 3 155 983
- RO-A2- 133 952
- MEHRAN GHASEMLOU ET AL: "Development and characterisation of a new biodegradable edible film made from kefiran, an exopolysaccharide obtained from kefir grains", FOOD CHEMISTRY, ELSEVIER LTD, NL, vol. 127, no. 4, 1 February 2011 (2011-02-01), pages 1496 - 1502, XP028371789, ISSN: 0308-8146, [retrieved on 20110206], DOI: 10.1016/J.FOODCHEM.2011.02.003
- EXARHOPOULOS STYLIANOS ET AL: "Biodegradable Films from Kefiran-Based Cryogel Systems", MACROMOL, vol. 2, no. 3, 13 July 2022 (2022-07-13), pages 324 - 345, XP093116504, ISSN: 2673-6209, DOI: 10.3390/macromol2030021

## Description

The subject of the invention is the method of preparing kefiran foil doped with violacein and kefiran foil for use as / in wound dressing materials and biomaterials.

Kefiran is an exopolysaccharide obtained from microorganisms present in kefir grains. It is synthesized by a few *Lactobacillus* species, e.g. *L. kefiranofaciens, L. kefirgranum, L. parakefir, L. kefir, L. delbrueckii subsp. Bulgaricus.* Due to good mechanical and protective properties of kefiran, it is applied for producing packaging foils, but it can also be used as a food additive. Moreover, the unique properties of kefiran, such as antibacterial, anti-inflammatory, antioxidant, and biocidal properties make it a good candidate for dressing matrix.

They have been discussed in the research paper entitled *"*Synthesis and characterization of biocompatible methacrylated kefiran hydrogels: Towards tissue engineering applications ", Radhouani et al., 2021. According to the authors of the paper, the possibilities of hydrogel applications are still limited, mainly due to their low mechanical strength and brittleness. Therefore, in order to improve their properties, certain physical and chemical crosslinking modifications are implemented. In the above mentioned research, methacrylated kefiran was synthesized in the reaction between Kefiran and methacrylate anhydride (MA). The developed MA-Kefiran was physicochemically characterized and its biological properties were examined with a variety of methods. The chemical modification of MA-Kefiran was confirmed with the use of 1H-NMR i FTIR, and GPC-SEC showed the average Mw 793kDa (PDI 1.3). The collected mechanical data exhibited that MA-Kefiran is a pseudoplastic fluid with the extrusion force of 11.21 2.87N. Additionally, MA-Kefiran 3D cryogels were successfully developed and fully characterized. Owing to micro-CT i SEM, scaffolds presented high porosity (85.53 ± 0.15%) and pore size (33.67 ± 3.13m), thick walls of the pores (11.92 ± 0.44m) as well as uniform structure. MA-Kefiran turned out to be biocompatible since it does not present a hemolytic activity and shows high viability against L929 cells, tested by with AlamarBlue^{®} assay. By attaching methacrylate groups into the kefiran polysaccharide chain, the MA-Kefiran product in the form of a hydrogel was developed. The product is applicable in tissue engineering and regenerative medicine.

In research paper entitled "Development and characterisation of a new biodegradable edible film made from kefiran, an exopolysaccharide obtained from kefir grains" M. Ghasemlou, F. Khodaiyan, A. Oromiehie, M. Saeid Yarmand, Food Chemistry, Elsevier Ltd, NL, vol. 127, no. 4, 1 February 2011, pages 1496-1502 it is disclosed a method to develop a biodegradable edible film made from kefiran, an exopolysaccharide obtained from kefir grains. The given paper does not disclose a method of preparing a kefiran foil including violacein and cannabis oil.

In research paper entitled "Biodegradable Films from Kefiran-Based Cryogel Systems" S. Exarhopoulos, A. Goulas, G. Dimitreli, Macromol, vol. 2, no. 3, 13 July 2022 (2022-07-13), pages 324-345 it is disclosed a method to prepare a biodegradable film from kefiran-based cryogel system, which may find application in the biotechnology, the medicine, the pharma, and the cosmetic industries.

The method comprises the kefiran extraction and purification. The research paper does not disclose a methods of preparing a kefiran foil including violacein and cannabis oil.

Since it is caused, each wound is prone to some microbe infection. Therefore, a kefiran matrix was enriched with violacein, an antibacterial substance and a natural dye produced by the *Chromobacterium violaceum* bacteria giving a characteristic purple-violet color. Violacein is used as an insecticide (patent description EP 2632272) and a substance protecting against infestation with pests (patent description EP 2770836). Its use as a foil component has not been reported.

From Romanian patent application RO 133952 A2 it is known a process for obtaining microporous collagen polysaccharide matrices, containing violacein, intended for biomedical and dermato-cosmetic applications, characterized in that it provides sterile porous solid substrates, with controlled hydrophilicity, hypo-immunogenic and hypo-allergenic, with the role of disposable dressing or compress. Violacein gives the matrix a narrow-spectrum antibacterial role and induces an echo-immunomodulatory effect in local inflammatory mechanisms. The given patent application does not disclose a kefiran foil, nor a method of preparing kefiran foil including violacein and cannabis oil.

The Canadian patent application CA 3 155 983 A1 discloses a medicinal, cosmetic or dermatologic product comprising: a sheet-like layer comprising a reconstituted cannabis material, the sheet-like layer having a shape configured to cover a portion of a users body (claim 1). The product may be a wound dressing (paragraphs [0017]). The given patent application does not disclose a kefiran foil, nor a method of preparing a kefiran foil including violacein and cannabis oil.

The purpose of the invention is to develop the method of preparing kefiran materials which will allow their use as wound dressing materials and biomaterials, and thus, the application of kefiran as a polymer substrate and matrix in wound dressing materials and biomaterials as well as the use of violacein as an active substance in wound dressing materials and biomaterials.

The subject of the invention is the method of producing kefiran foil for use as / in wound dressing materials and biomaterials characteristic in that kefiran is dissolved in deionized water at the temperature range of 90 - 100°C in the amount of 0.1g - 10g preferably 0.2g of kefiran per 10mL deionized water, mixed with a stirrer preferably a magnetic stirrer, cooled, violacein is added in the amount of 0.05mg - 0.2mg, preferably 0.1mg of violacein per 1mL of the mixture, mixed with a stirrer preferably a magnetic stirrer, degasified in a vacuum desiccator, cannabis oil is added in the amount of 0.05mg - 0.2mg preferably 0.1mg of cannabis oil per 1mL of the mixture, plant glycerin is added in the amount of 0.01g - 0.05g preferably 0.03g glycerin per 1mL of the mixture, mixed, and the thus produced homogeneous mixture is poured on a Petri dish and left until the foil is formed. Preferably kefiran was isolated from Tibetan mushrooms. Preferably kefiran is mixed with deionized water with a stirrer preferably a magnetic stirrer preferably at the rate of 400rpm - 800rpm to most preferably 600rpm, at the temperature of 90°C - 100°C preferably 100°C for 0.2h - 1.5h preferably 1h. Preferably cooling proceeds until the temperature of the mixture reaches 18°C - 25°C, preferably 22°C. Preferably the mixture with violacein is mixed with a stirrer preferably a magnetic stirrer preferably at the rate of 300rpm - 800rpm most preferably 600rpm, at the temperature of the mixture of 18°C - 25°C, preferably 22°C for 0.2h - 1.5h preferably 30min. Preferably degasification is carried out in a vacuum desiccator at the temperature of 40°C - 60°C preferably 40°C for 0.2h - 1.5h preferably1h. Preferably the mixture with glycerin is mixed with a stirrer preferably a magnetic stirrer preferably at the rate of 300rpm - 800rpm most preferably at 600rpm, at the temperature of 18°C - 25°C of the mixture, preferably 22°C for 0.2h - 1.5h preferably 30min. Preferably a plastic or glass Petri dish is used, preferably of 30mm - 60mm in diameter, most preferably 45mm. Preferably the formation of foil is carried out in a dry place without access to light, preferably for 24h - 64h most preferably for 48h.

The subject of the invention is also kefiran foil for use as / in wound dressing materials and biomaterials characterized in that it contains kefiran in the amount of 30% - 40 %, violacein in the amount of 3% - 4%, cannabis oil in the amount of 3% - 4%, glycerin in the amount of 1% - 4%, and deionized water to complete.

The use of exclusively natural products to manufacture the dressing is an advantage of the invention as the material is safe and ecologically friendly. Natural active substances were applied, and the resulting properties of the material support wound healing (including antibacterial, pain relieving, anti-inflammatory, regenerative activity), without causing side-effects which often occurs when synthetic antibiotics are used. Also, the materials are easily accessible and relatively cost-efficient in production.

The subject of the invention was disclosed in the examples of performance presented below.

### Example I.

In the example of preparation, kefiran individually isolated from Tibetan mushrooms according to the procedure described by Rodrigues et al. was applied (Kamila Leite Rodrigues, Luc'elia Rita Gaudino Caputo, Jose Carlos Tavares Carvalho, Jo~ao Evangelista, Jose Maur' 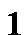 cio Schneedorf. Antimicrobial and healing activity of kefir and kefiran extract. International Journal of Antimicrobial Agents 25 (2005) 404-408.). In addition to kefiran, violacein was also used.

In order to prepare kefiran foil doped with violacein, freshly isolated kefiran was dissolved in hot deionized water (C=2% w/w) at the temperature of 90°C in the amount of 0.2g per 10mL of water and mixed with the use of a magnetic stirrer at the rate of 600rpm, at the temperature of 100°C for 1h.

Next, the solution was cooled to room temperature 22°C, and then violacein was added (C=1%, 1mL) in the amount of 0.1mg per 1mL of the solution. The whole content was mixed at room temperature 22°C for 30min with a magnetic stirrer at the rate of 600rpm. The solution was degasified in the vacuum desiccator at the temperature of 40°C for 1h. Next cannabis oil was added in the amount of 0.1mg of cannabis oil per 1mL of the solution and plant glycerin in the amount of 0.03g per 1mL of the solution as a plasticizer, and stirring was continued for 30 min at the rate of 600rpm at room temperature 22°C. The thus produced homogeneous mixture was poured on a plastic Petri dish of 45 mm in diameter and left in a dry and dark place for 48h till the solvent evaporation and the foil formation.

As the result of the example preparation, kefiran foil characterized by good physicochemical and antibacterial properties and the following composition was formed:
- kefiran in the amount of 35%
- violacein in the amount of 3.5%
- cannabis oil in the amount of 4%
- plant glycerin in the amount of 2.5 %
- the remaining content - deionized water

### ATR-IR analysis:

The bands at 2921 cm⁻¹ indicate the occurrence of C-H vibrations in the kefiran saccharide chain, which can be attributed to the presence of methyl and methylene groups. The band at 1408 cm⁻¹ relates to the presence of CH₂ and OH groups. The bands in the range 1000-1200 cm⁻¹ indicate the presence of C-O-C and C-O bonds resulting from kefiran alcohol groups. In the spectrum, the bands proving the presence of violacein in the prepared film also appear. The wide band at 3341 cm⁻¹ results from the presence of NH groups of the indole core whereas the band at 1651 cm⁻¹ corresponds to the carbonyl group present in the violacein molecule.

In other examples of preparation kefiran isolated from Iranian or Italian kefir grains can be applied.

### Example II.

In the example of preparation, kefiran individually isolated from Tibetan mushrooms according to the procedure described by Rodrigues et al. was applied (Kamila Leite Rodrigues, Luc'elia Rita Gaudino Caputo, Jose Carlos Tavares Carvalho, Jo~ao Evangelista, Jose Maur' 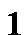 cio Schneedorf. Antimicrobial and healing activity of kefir and kefiran extract. International Journal of Antimicrobial Agents 25 (2005) 404-408.). In addition to kefiran, violacein was also used.

In order to prepare kefiran foil doped with violacein, freshly isolated kefiran was dissolved in hot deionized water (C=2% w/w) at the temperature of 95°C in the amount of 5g per 10mL of water and mixed with the use of a magnetic stirrer at the rate of 400 rpm, at the temperature of 95°C for 0.02h.

Next, the solution was cooled to room temperature 18°C, and then violacein was added (C=1%, 1mL) in the amount of 0.05mg per 1mL of the solution. The whole content was mixed at room temperature 18°C for 0.2h with a magnetic stirrer at the rate of 400 rpm at room temperature. The solution was degasified in the vacuum desiccator at the temperature of 50°C for 0.2h. Next cannabis oil was added in the amount of 0.05mg of cannabis oil per 1mL of the solution and plant glycerin in the amount of 0.01g per 1mL of the solution as a plasticizer, and stirring was continued for 0.2h at the rate of 300 rpm at room temperature 18°C. The thus produced homogeneous mixture was poured on a plastic Petri dish of 30 mm in diameter and left in a dry and dark place for 64h till the solvent evaporation and the foil formation.

As the result of the example preparation, kefiran foil characterized by good physicochemical and antibacterial properties and the following composition was formed:
- kefiran in the amount of 30%
- violacein in the amount of 3%
- cannabis oil in the amount of 3.5%
- plant glycerin in the amount of 1%
- the remaining content - deionized water

In other examples of preparation kefiran isolated from Iranian or Italian kefir grains can be applied.

### Example III.

In the example of preparation, kefiran individually isolated from Tibetan mushrooms according to the procedure described by Rodrigues et al. was applied (Kamila Leite Rodrigues, Luc'elia Rita Gaudino Caputo, Jose Carlos Tavares Carvalho, Jo^{~}ao Evangelista, Jose Maur' 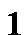 cio Schneedorf. Antimicrobial and healing activity of kefir and kefiran extract. International Journal of Antimicrobial Agents 25 (2005) 404-408.). In addition to kefiran, violacein purchased from Immuno-Life company was also used.

In order to prepare kefiran foil doped with violacein, freshly isolated kefiran was dissolved in hot deionized water (C=2% w/w) at the temperature of 100°C in the amount of 10g per 10mL of water and mixed with the use of a magnetic stirrer at the rate of 800 rpm, at the temperature of 90°C for 1.5h.

Next, the solution was cooled to room temperature 25°C, and then violacein was added (C=1%, 1mL) in the amount of 0.2mg per 1mL of the solution. The whole content was mixed at room temperature 25°C for 1.5h with a magnetic stirrer at the rate of 800 rpm at room temperature. The solution was degasified in the vacuum desiccator at the temperature of 60°C for 1.5h. Next cannabis oil was added in the amount of 0.2mg of cannabis oil per 1mL of the solution and plant glycerin in the amount of 0.05g per 1mL of the solution as a plasticizer, and stirring was continued for 1.5h at the rate of 800 rpm at room temperature 25°C. The thus produced homogeneous mixture was poured on a plastic Petri dish of 60 mm in diameter and left in a dry and dark place for 24h till the solvent evaporation and the foil formation.

As the result of the example preparation, kefiran foil characterized by good physicochemical and antibacterial properties and the following composition was formed:
- kefiran in the amount of 40%
- violacein in the amount of 4%
- cannabis oil in the amount of 3%
- plant glycerin in the amount of 4%
- the remaining content - deionized water

In other examples of preparation kefiran isolated from Iranian or Italian kefir grains can be applied.

## Claims

1. Method of preparing kefiran foil for use as / in wound dressing materials and biomaterials **characterized in that** kefiran is dissolved in deionized water at the temperature of 90°C - 100°C in the amount of 0.1g - 10g preferably 0.2g kefiran per 10mL of deionized water, mixed with a stirrer preferably magnetic stirrer, cooled, violacein is added in the amount of 0.05mg - 0.2mg preferably 0.1mg violacein per 1mL of the mixture, mixed with a stirrer preferably magnetic stirrer, degasified in a vacuum desiccator, cannabis oil is added in the amount 0.05mg - 0.2mg preferably 0.1mg of cannabis oil per 1mL of the mixture, plant glycerin is added in the amount 0.01g - 0.05g preferably 0.03g of glycerin per 1mL of the mixture, mixed and the thus prepared homogeneous mixture is poured on a Petri dish and left till the foil formation.

2. Method according to claim 1 **characterized in that** kefiran was isolated from Tibetan mushrooms.

3. Method according to claim 1 or 2 **characterized in that** kefiran is mixed with deionized water with a stirrer preferably magnetic stirrer preferably at the rate of 400rpm - 800rpm most preferably 600rpm, at the temperature of 90°C - 100°C preferably 100°C for 0.2 - 1.5h preferably 1h.

4. Method according to claim 1,2 or 3 **characterized in that** cooling is carried out till the temperature of the mixture is at 18°C - 25°C preferably 22°C.

5. Method according to claim 1,2,3 or 4 **characterized in that** the mixture with violacein is mixed with a stirrer preferably magnetic stirrer preferably at the rate of 300rpm - 800rpm most preferably 600rpm, at the temperature of 18°C - 25°C of the mixture preferably 22°C for 0.2 - 1.5h preferably 30min.

6. Method according to claim 1,2,3,4 or 5 **characterized in that** degasification is carried out in a vacuum desiccator at the temperature 40°C - 60°C preferably 40°C for 0.2 - 1.5h preferably 1h.

7. Method according to claim 1,2,3,4,5 or 6 **characterized in that** the mixture with glycerin is mixed with a stirrer preferably magnetic stirrer preferably at the rate of 300rpm - 800rpm most preferably 600rpm, at the temperature of 18°C - 25°C of the mixture preferably 22°C for 0.2 - 1.5h preferably 30min.

8. Method according to claim 1,2,3,4,5,6 or 7 **characterized in that** a Petri dish made of plastic or glass is used preferably of 30mm - 60mm in diameter most preferably 45mm.

9. Method according to claim 1,2,3,4,5,6,7 or 8 **characterized in that** the formation of foil is carried out in a dry place without access of light, preferably for 24h - 64h most preferably 48h.

10. Kefiran foil for use as / in wound dressing materials and biomaterials **characterized in that** it contains kefiran in the amount of 30% - 40%, violacein in the amount of 3% - 4%, cannabis oil in the amount of 3% - 4%, glycerin in the amount of 1% - 4 %, the remaining content is deionized water.

## Patentansprüche

1. Verfahren zur Herstellung von Kefiranfolie zur Anwendung als/in Verbandsmaterialien und Biomaterialien, **gekennzeichnet dadurch, dass** Kefiran in deionisiertem Wasser mit einer Temperatur von 90 - 100 °C in einer Menge von 0,1 g - 10 g, vorzugsweise 0,2 g Kefiran pro 10 ml deionisiertem Wasser gelöst wird, mit einem Mischer, vorzugsweise einem Magnetmischer, gemischt wird, abgekühlt wird, Violacein in einer Menge von 0,05 mg - 0,2 mg, vorzugsweise 0,1 mg Violacein pro 1 ml Gemisch hinzugefügt wird, mit einem Mischer, vorzugsweise einem Magnetmischer, gemischt wird, in einem Vakuumtrockner entgast wird, Hanföl in einer Menge von 0,05 mg - 0,2 mg, vorzugsweise 0,1 mg Hanföl pro 1 ml Gemisch hinzugefügt wird, pflanzliches Glycerin in einer Menge von 0,01 g - 0,05 g, vorzugsweise 0,03 g Glycerin pro 1 ml Gemisch hinzugefügt wird, gemischt wird, und das auf die Weise erhaltene homogene Gemisch in eine Petrischale gefüllt und bis zum Formen einer Folie belassen wird.

2. Das Verfahren nach Anspruch 1, **gekennzeichnet dadurch, dass** das Kefiran aus einem tibetischen Kefirpilz gewonnen wurde.

3. Das Verfahren nach Anspruch 1 oder 2, **gekennzeichnet dadurch, dass** das Kefiran mit deionisiertem Wasser mit einem Mischer, vorzugsweise einem Magnetmischer, gemischt wird, vorzugsweise mit einer Geschwindigkeit von 400 - 800 U/Min., besonders bevorzugt bis 600 U/Min., bei einer Temperatur von 90 °C - 100 °C, vorzugsweise 100 °C, während einer Zeit von 0,2 - 1,5 h, vorzugsweise 1 h.

4. Das Verfahren nach Anspruch 1, 2 oder 3, **gekennzeichnet dadurch, dass** das Abkühlen bis zum Erreichen einer Temperatur von 18 °C - 25 °C des Gemisches, vorzugsweise 22 °C, durchgeführt wird.

5. Das Verfahren nach Anspruch 1, 2, 3 oder 4, **gekennzeichnet dadurch, dass** das Gemisch mit Violacein mit einem Mischer, vorzugsweise einem Magnetmischer, gemischt wird, vorzugsweise mit einer Geschwindigkeit von 300 - 800 U/Min., besonders bevorzugt 600 U/Min., bei einer Temperatur von 18 °C - 25 °C, vorzugsweise 22 °C, während einer Zeit von 0,2 - 1,5 h, vorzugsweise 30 Min.

6. Das Verfahren nach Anspruch 1, 2, 3, 4 oder 5, **gekennzeichnet dadurch, dass** das Entgasen in einem Vakuumtrockner durchgeführt wird, bei einer Temperatur von 40 - 60 °C, vorzugsweise 40 °C, während einer Zeit von 0,2 - 1,5 h, vorzugsweise 1 h.

7. Das Verfahren nach Anspruch 1, 2, 3, 4, 5 oder 6, **gekennzeichnet dadurch, dass** das Gemisch mit Glycerin mit einem Mischer, vorzugsweise einem Magnetmischer, gemischt wird, vorzugsweise mit einer Geschwindigkeit von 300 - 800 U/Min., besonders bevorzugt 600 U/Min., bei einer Temperatur von 18 °C - 25 °C des Gemisches, vorzugsweise 22 °C, während einer Zeit von 0,2 - 1,5 h, vorzugsweise 30 Min.

8. Das Verfahren nach Anspruch 1, 2, 3, 4, 5, 6 oder 7, **gekennzeichnet dadurch, dass** eine Petrischale aus Kunststoff oder Glas verwendet wird, vorzugsweise mit einem Durchmesser von 30 bis 60 mm, besonders bevorzugt 45 mm.

9. Das Verfahren nach Anspruch 1, 2, 3, 4, 5, 6, 7 oder 8, **gekennzeichnet dadurch, dass** das Formen der Folie an einem trockenen Ort ohne Zugang zu Licht erfolgt, vorzugsweise während einer Zeit von 24 - 64 h, besonders bevorzugt 48 h lang.

10. Kefiranfolie zur Anwendung als/in Verbandsmaterialien und Biomaterialien, **gekennzeichnet dadurch, dass** sie Kefiran in einer Menge von 30 - 40 %, Violacein in einer Menge von 3 % - 4 %, Hanföl in einer Menge von 3 % - 4 %, Glycerin in einer Menge von 1 % - 4 % enthält und der Rest deionisiertes Wasser darstellt.

## Revendications

1. Procédé de fabrication d'un film de kéfiran destiné à être utilisé dans/comme des matériaux de pansement et des biomatériaux, **caractérisé en ce que** le kéfiran est dissous dans de l'eau déionisée à une température comprise entre 90 et 100 °C, à raison de 0,1 g à 10 g, de préférence 0,2 g de kéfiran pour 10 ml d'eau déionisée, mélangé à l'aide d'un agitateur, de préférence magnétique et refroidi, ensuite on ajoute de la violacéine en quantité comprise entre 0,05 mg à 0,2 mg, de préférence 0,1 mg de violacéine pour 1 ml de mélange, on mélange à l'aide d'un agitateur, de préférence magnétique, on dégazéifie dans un séchoir sous vide, on ajoute de l'huile de chanvre en quantité de 0,05 mg à 0,2 mg, de préférence 0,1 mg d'huile de chanvre pour 1 ml de mélange, on ajoute de la glycérine végétale en quantité comprise entre 0,01 g et 0,05 g, de préférence 0,03 g de glycérine par 1 ml de mélange, on mélange, puis on verse le mélange homogène ainsi obtenu dans une boîte de Pétri et on laisse reposer jusqu'à la formation d'un film.

2. Procédé selon la revendication 1, **caractérisé en ce que** le kéfiran a été isolé à partir d'un champignon tibétain.

3. Procédé selon la revendication 1 ou 2, **caractérisé en ce que** le kéfiran et l'eau déionisée sont mélangés à l'aide d'un agitateur, de préférence magnétique, à une vitesse comprise entre 400 et 800 tr/min, de préférence 600 tr/min, à une température comprise entre 90 °C et 100 °C, de préférence à 100 °C, pendant une durée de 0,2 à 1,5 heure, de préférence 1 heure.

4. Procédé selon la revendication 1, 2 ou 3, **caractérisé en ce que** le refroidissement est poursuivi jusqu'à ce que le mélange atteigne une température comprise entre 18 °C et 25 °C, de préférence 22 °C.

5. Procédé selon la revendication 1, 2, 3 ou 4, **caractérisé en ce que** le mélange contenant de la violacéine est agité à l'aide d'un agitateur, de préférence magnétique, à une vitesse comprise entre 300 et 800 tr/min, de préférence 600 tr/min, à une température comprise entre 18 °C et 25 °C, de préférence à 22 °C, pendant une durée de 0,2 à 1,5 heure, de préférence 30 min.

6. Procédé selon la revendication 1, 2, 3, 4 ou 5, **caractérisé en ce que** le dégazage est effectué dans un séchoir sous vide à une température comprise entre 40 et 60 °C, de préférence 40 °C, pendant une durée comprise entre 0,2 et 1,5 heure, de préférence 1 heure.

7. Procédé selon la revendication 1, 2, 3, 4, 5 ou 6, **caractérisé en ce que** le mélange avec la glycérine est agité à l'aide d'un agitateur, de préférence magnétique, de préférence à une vitesse comprise entre 300 et 800 tr/min, de préférence 600 tr/min, à une température du mélange comprise entre 18 °C et 25 °C, de préférence 22 °C, pendant une durée comprise entre 0,2 et 1,5 heure, de préférence 30 min.

8. Procédé selon les revendications 1, 2, 3, 4, 5, 6 ou 7, **caractérisé en ce qu'**une boîte de Pétri en plastique ou en verre est utilisée, de préférence d'un diamètre compris entre 30 et 60 mm, de préférence de 45 mm.

9. Procédé selon les revendications 1, 2, 3, 4, 5, 6, 7 ou 8, **caractérisé en ce que** la formation du film s'effectue dans un endroit sec à l'abri de la lumière, de préférence pendant une durée comprise entre 24 et 64 heures, de préférence pendant 48 heures.

10. Film de kéfiran destiné à être utilisé dans/comme des matériaux de pansement et des biomatériaux, **caractérisé en ce qu'**il contient du kéfiran à raison de 30 à 40 %, de la violacéine à raison de 3 à 4 %, de l'huile de chanvre à raison de 3 à 4 %, de la glycérine à raison de 1 à 4 %, le reste étant constitué d'eau déionisée.
